# FASCICULE DE BREVET EUROPEEN

(11) **EP 2 266 531 B1**
(45) Date de publication et mention de la délivrance du brevet: **14.06.2017**
(21) Numéro de dépôt: 10166606.3
(22) Date de dépôt: 21.06.2010
(51) Int. Cl.: A61K 8/81, A61Q 19/00, A61K 8/35, A61K 8/37, A61K 8/40, A61K 8/46, A61K 8/49, A61Q 17/04, A61K 8/02

(54) **Composition cosmétique comprenant un polymère superabsorbant et un filtre uv organique**
Kosmetische Zusammensetzung enthaltend ein superabsorbierendes Polymer und einen organischen UV-Filter
Cosmetic composition comprising a superabsorbent polymer and an organic UV filter

(30) Priorité: 24.06.2009 FR 0954311
(43) Date de publication de la demande: 29.12.2010
(73) Titulaire: L'Oréal, 75008 Paris (FR)
(72) Inventeur: Lorant, Raluca, 94320, Thiais (FR); Fageon, Laure, 75013, Paris (FR); Boutelet, Karl, 75011, Paris (FR)
(74) Mandataire: Duvert, Sandra

(56) Documents cités:
- EP-A2- 1 952 842
- FR-A1- 2 874 174

## Description

La présente demande se rapporte à une composition sous forme d'émulsion comprenant un polymère superabsorbant et un filtre UV organique, et à l'utilisation de la dite composition dans les domaines cosmétique et dermatologique, en particulier pour le soin, le traitement des matières kératiniques.
Les compositions antisolaires se présentent souvent sous la forme d'une émulsion de type huile-dans-eau, ou eau-dans-huile, de gels ou de produits anhydres qui contiennent, à des concentrations diverses, un ou plusieurs filtres organiques et/ou inorganiques, insolubles et/ou solubles, lipophiles et/ou hydrophiles, capables d'absorber sélectivement le rayonnement UV nocif. Ces filtres et leurs quantités étant sélectionnés en fonction de l'indice de protection recherché. Selon leur caractère lipophile ou au contraire hydrophile, ces filtres peuvent se répartir, respectivement, soit dans la phase grasse, soit dans la phase aqueuse, de la composition finale.

Les filtres organiques sont couramment utilisés dans les formulations solaires. Cependant pour un certain nombre d'entre eux, en particulier les filtres lipophiles, leur incorporation dans des émulsions entraîne une dégradation du toucher cosmétique, en particulier un effet collant lors de l'application sur la peau puis après pénétration du produit. Cet effet collant rend l'utilisation du produit désagréable pour l'utilisateur, et ce désagrément s'accentue d'autant plus lorsque ces produits sont utilisés en période de forte chaleur.

Pour pallier aux problèmes de collant de compositions cosmétiques comprenant des actifs lipophiles à motifs sucres, il a été proposé des élastomères siliconés permettant de réduire l'effet collant. Toutefois, cette solution n'est pas envisageable dans le cas des filtres liposolubles en raison de leur incompatibilité avec les composés siliconés.

L'objectif de l'invention est de pouvoir réaliser des émulsions présentant de bonnes propriétés cosmétiques sans avoir les inconvénients de l'art antérieur.

La demanderesse a découvert que l'utilisation d'un polymère superabsorbant en association avec un filtre UV organique permettait de réduire voir éliminer l'effet collant et permettait en particulier de réaliser des compositions cosmétiques et notamment des émulsions ayant de très bonnes propriétés cosmétiques, en particulier de douceur et de glissant à l'application sur la peau, même en présence d'un taux élevé de filtres UV lipophiles..

La présente invention a ainsi pour objet une composition sous forme d'émulsion comprenant au moins une phase aqueuse et au moins une phase grasse, un polymère superabsorbant et au moins un filtre UV organique.

La composition de l'invention étant destinée notamment à une application topique comprend un milieu physiologiquement acceptable, c'est-à-dire un milieu compatible avec toutes les matières kératiniques telles que la peau, les ongles, les muqueuses et les fibres kératiniques (telles que les cheveux, les cils).

La composition obtenue selon l'invention présente l'avantage d'avoir une texture homogène, très douce, non collante et fraîche à l'application, donc très agréable à utiliser.

L'invention a également pour objet un procédé de traitement cosmétique des matières kératiniques consistant à appliquer sur les matières kératiniques une composition telle que définie plus haut.

### Polymère superabsorbant

On entend par « polymère superabsorbant », un polymère qui est apte à son état sec à absorber spontanément au moins 20 fois son propre poids de fluide aqueux, en particulier d'eau et notamment d'eau distillée. De tels polymères super absorbants sont décrits dans l'ouvrage "Absorbent polymer technology, Studies in polymer science 8" de L. BRANNON-PAPPAS et R. HARLAND, édition Elsevier, 1990.
Ces polymères ont une grande capacité d'absorption et de rétention de l'eau et de fluides aqueux. Après absorption du liquide aqueux, les particules du polymère ainsi imbibées de fluide aqueux restent insolubles dans le fluide aqueux et conservent ainsi leur état particulaire individualisé.
Le polymère superabsorbant peut avoir une capacité d'absorption d'eau allant de 20 à 2000 fois son propre poids (soit 20 g à 2000 g d'eau absorbée par gramme de polymère absorbant), de préférence de 30 à 1500 fois, et mieux de 50 à 1000 fois. Ces caractéristiques d'absorption d'eau sont définies aux conditions normales de température
(25 °C) et de pression (760 mm Hg soit 100000 Pa) et pour de l'eau distillée.

La valeur de la capacité d'absorption d'eau d'un polymère peut être déterminée en dispersant 0,5 g de polymère(s) dans 150 g d'une solution d'eau, en attendant 20 minutes, en filtrant la solution non absorbée sur un filtre de 150 µm pendant 20 minutes et en pesant l'eau non absorbée.

Le polymère superabsorbant utilisé dans la composition de l'invention se présente sous forme de particules, qui, une fois hydratées, gonflent en formant des billes molles ayant un diamètre moyen en inférieure ou égale à 100 µm, de préférence inférieure ou égale à 50 µm, allant par exemple de 10 à 100 µm.

Les polymères superabsorbants utilisés dans la présente invention se présentent sous forme de particules sphériques.

Les polymères superabsorbants utilisés dans la présente invention sont des polyacrylates de sodium réticulés comme par exemple ceux commercialisés sous les dénominations Octacare X100, X110 et RM100 par la société Avecia, ceux commercialisés sous les dénominations Flocare GB300 et le Flosorb 500 par la société SNF, ceux commercialisés sous les dénominations Luquasorb 1003, Luquasorb 1010, Luquasorb 1280 et Luquasorb 1110 par la société BASF, ceux commercialisés sous les dénominations Water Lock G400 et G430 (nom INCl: Acrylamide/Sodium acrylate copolymer) par la société Grain Processing, ou encore l' AQUA KEEP 10 SH NF proposé par la société Sumitomo Seika. polymères ont de préférence une capacité d'absorption d'eau de 10 à 100 g/g, de préférence de 20 à 80 g/g et mieux de 50 à 70 g/g.
Le polymère superabsorbant peut être présent dans la composition de l'invention en une teneur en matière active allant par exemple de 0,03 à 15 % en poids, de préférence de 0,05 à 10% en poids, de préférence allant de 0,1 à 5 % en poids, préférentiellement allant de 0,1 à 3%, voire de 0,1 à 2 % en poids par rapport au poids total de la composition.

### Filtres organiques

Les filtres organiques sont généralement présents dans les compositions selon l'invention dans des proportions allant de 0,05 à 30% en poids par rapport au poids total de la composition, de préférence allant de 0,1 à 20% en poids, et mieux de 0,5 à 15% en poids par rapport au poids total de la composition.
Les filtres organique peuvent être choisis parmi les filtres organiques lipophiles ou hydrophiles, ou leurs mélanges.
Par « filtre lipophile» on entend tout filtre susceptible d'être complètement dissous à l'état moléculaire dans une phase grasse liquide ou bien d'être solubilisé sous forme colloïdale (par exemple sous forme micellaire) dans une phase grasse liquide.
Par « filtre UV hydrophile», on entend tout agent filtrant les radiations UV susceptible d'être complètement dissous à l'état moléculaire dans la phase aqueuse de l'émulsion ou bien d'être solubilisé sous forme colloïdale (par exemple sous forme micellaire) dans la phase aqueuse de l'émulsion.

De préférence, la composition comprend au moins un filtre organique lipophile.

Les filtres organiques lipophiles peuvent être choisis parmi les dérivés de l'acide para-aminobenzoique, les dérivés salicyliques, les dérivés cinnamiques, les benzophénones et aminobenzophénones, les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines, les dérivés de diphényl butadiène malonates ou malonitriles, les chalcones et leurs mélanges.

Parmi les filtres UVA lipophiles capables d'absorber les UV de 320 à 400 nm, on peut citer

### Les dérivés du dibenzoylméthane :

- le 4-isopropyl-dibenzoylméthane, vendu sous la dénomination de "EUSOLEX 8020" par la Société MERCK, et répondant à la formule suivante : 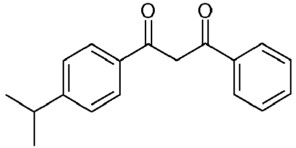
- le 1-(4-methoxy-1-benzofuran-5-yl)-3-phenylpropane-1,3-dione, proposé à la vente par la société QUEST sous le nom de Pongamol de formule : 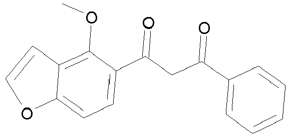
- le 1-(4-tert-butylphenyl)-3-(2-hydroxyphenyl)propane-1,3-dione de formule : 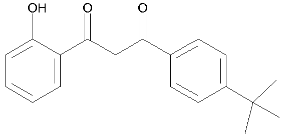
- le Butyl Methoxydibenzoylmethane vendu notamment sous le nom commercial « PARSOL 1789 » par HOFFMANN LA ROCHE,

### Les aminobenzophénones

2-(4-diethylamino-2-hydroxybenzoyl)-benzoate de n-hexyle vendu sous le nom commercial« UVINUL A +»

### Les dérivés anthraniliques :

Menthyl anthranilate vendu sous le nom commercial « NEO HELIOPAN MA » par HAARMANN et REIMER,

### Les dérivés de 4,4-diarylbutadiène :

1,1-dicarboxy (2,2'-diméthyl-propyl)-4,4-diphénylbutadiène
Les préférentiels sont :
Parmi les filtres UVB lipophiles capables d'absorber les UV de 280 à 320 nm, on peut citer

### Les para-aminobenzoates :

Ethyl PABA
Ethyl Dihydroxypropyl PABA
Ethylhexyl Dimethyl PABA (ESCALOL 507 de ISP)

### Les dérivés salicyliques :

- Homosalate vendu sous le nom « Eusolex HMS » par Rona/EM Industries,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
   TEA Salicylate, vendu sous le nom « NEO HELIOPAN TS » par Haarmann et REIMER,

### Les cinnamates

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Diisopropyl Methylcinnamate,
Cinoxate,
Glyceryl Ethylhexanoate Diméthoxycinnamate

### Les dérivés de β,β'-diphénylacrylate :

Octocrylène, vendu notamment sous le nom commercial « UVINUL N539 » par BASF, Etocrylène, vendu notamment sous le nom commercial « UVINUL N35 » par BASF,

### Les dérivés du benzylidène camphre :

3-Benzylidène camphre fabriqué sous le nom « MEXORYL SD » par CHIMEX, Méthylbenzylidène camphre vendu sous le nom « EUSOLEX 6300 » par MERCK, Polyacrylamidométhyle Benzylidène Camphre fabriqué sous le nom « MEXORYL SW » par CHIMEX,

### Les dérivés de triazine :

Ethylhexyl triazone vendu notamment sous le nom commercial « UVINUL T150 » par BASF,
Diethylhexyl Butamido Triazone vendu sous le nom commercial « UVASORB HEB » par SIGMA 3V,
2,4,6-tris(4'-amino benzalmalonate de dinéopentyle)-s-triazine
la 2,4,6-tris-(4'-amino benzalmalonate de diisobutyle)-s- triazine,
2,4-bis(4'-amino benzalmalonate de dinéopentyle)-6-(4'-aminobenzoate de n-butyle)-s-triazine,
2,4-bis(4'-amino benzoate de n-butyle)-6-(aminopropyltrisiloxane)-s-triazine,

### Les dérivés d'imidazolines :

Ethylhexyl Dimethoxybenzylidene Dioxoimidazoline Propionate,

### Les dérivés du benzalmalonate :

Polyorganosiloxanes à fonction benzalmalonate tels que le Polysilicone-15 vendu sous la dénomination commerciale « PARSOL SLX » par HOFFMANN LA ROCHE Di-neopentyl 4'-méthoxybenzalmalonate,

### Les dérivés de mérocyanine

Octyl-5-N,N-diethylamino-2-phenysulfonyl-2,4-pentadienoate

Parmi les filtres à spectre large lipophiles capables d'absorber les UVA et les UVB, on peut citer

### Les dérivés de benzophénone

Benzophenone-1 vendu sous le nom commercial « UVINUL 400 » par BASF, Benzophenone-2 vendu sous le nom commercial « UVINUL D50 » par BASF Benzophenone-3 ou Oxybenzone, vendu sous le nom commercial « UVINUL M40 » par BASF,
Benzophenone-6 vendu sous le nom commercial « Helisorb 11 » par Norquay Benzophenone-8 vendu sous le nom commercial « Spectra-Sorb UV-24 » par American Cyanamid
Benzophenone-10,
Benzophenone-11,
Benzophenone-12,

### Dérivés de benzotriazole :

Drometrizole Trisiloxane vendu sous le nom « Silatrizole » par RHODIA CHIMIE Bumetrizole vendu sous le nom TINOGUARD AS par CIBA-GEIGY

### Les dérivés bis-résorcinyl triazines

Bis-Ethylhexyloxyphénol Méthoxyphenyl Triazine vendu sous le nom commercial « TINOSORB S » par CIBA GEIGY,

### Les dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V

Les dérivés de la famille des diphényl butadiènes malonates ou malonitriles sont les dérivés de formule (IV) générale : 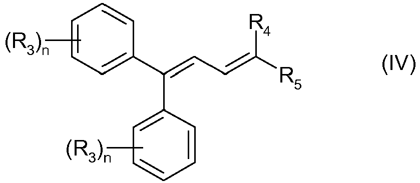
dans laquelle R₃ représente un groupement alkyle en C₁-C₂, un groupement alcoxy en C₁-C₂ et n est égal à 0, 1 ou 2 ;
R₄ et R₅, identiques ou différents représentent -COOR₆, -(C=O)NHR₆, -(C=O)R₆ , -CN dans lequel R₆ représente un groupement alkyle contenant de 1 à 12 atomes de carbone, linéaire ou ramifié et pouvant contenir des groupements silaniques, siloxaniques ou polysiloxaniques.

Parmi les dérivés de diphényl butadiène malonates ou malonitriles, on peut notamment citer, de manière non limitative :
- dimethyl 2-(3,3-diphenylprop-2-enylidene)malonate
- diisobutyl 2-(3,3-diphenylprop-2-enylidene)malonate
- bis(1,3-dimethylbutyl) 2-(3,3-diphenylprop-2-enylidene)malonate
- dineopentyl 2-(3,3-diphenylprop-2-enylidene)malonate
- methyl (2Z)-2-cyano-5,5-diphenylpenta-2,4-dienoate
- ethyl (trimethylsilyl)methyl (2Z)-2-(3,3-diphenylprop-2-enylidene)malonate
- (2E)-2-cyano-5,5-diphenyl-N-(3-{1,3,3,3-tetramethyl-1-[(trimethylsilyl)oxy]disiloxanyl}propyl)penta-2,4-dienamide
- ethyl 2-methyl-3-{1,3,3,3-tetramethyl-1-[(trimethylsilyl)oxy]disiloxanyl}propyl (2E)-2-(3,3-diphenylprop-2-enylidene)malonate
- ethyl (2Z)-5,5-diphenyl-2-{[(3-{1,3,3,3-tetramethyl-1-[(trimethylsilyl)oxy]disiloxanyl}propyl)amino]carbonyl}penta-2,4-dienoate

Parmi les dérivés de diphényl butadiènes mentionnés ci-dessus, on utilisera en particulier le dineopentyl 2-(3,3-diphenylprop-2-enylidene)malonate répondant à la formule suivante :

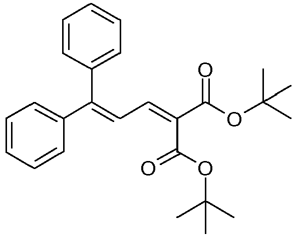

Il est connu d'utiliser ces dérivés de diphényl butadiènes dans des compositions solaires, le brevet EP 0916335 décrit les dérivés carbonés ainsi que ses modes d'obtention et les brevets EP 1535947 et EP 1535925 décrivent les dérivés siloxaniques et silaniques, respectivement.

Les dérivés de la famille des chalcones sont les dérivés de formule générale (V) suivante : 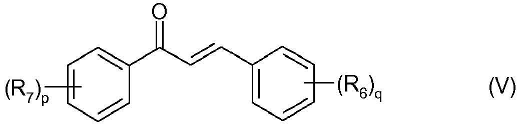 dans laquelle les radicaux R₆ et R₇ désignent indépendamment de chacun un atome d'hydrogène, le radical hydroxy, un groupement alkyle ou alcényle en C₁-C₁₂, linéaire ou ramifié, un groupement alcoxy en C₁-C₁₂, linéaire ou ramifié ou un groupe acyloxy en C₂-C₂₀, linéaire ou ramifié ; petq

Parmi les dérivés de chalcones, on peut notamment citer, de manière non limitative :
- hydroxy-2' chalcone
- hydroxy-4' chalcone
- méthoxy-4' chalcone
- hydroxy-2' méthoxy-4 chalcone
- hydroxy-2' hexyloxy-4 chalcone
- hydroxy-2' méthyl-4 chalcone
- hydroxy-2' hexyloxy-3 chalcone
- hydroxy-2' hexyloxy-4'méthyl-4 chalcone
- hydroxy-2' hexanoyloxy-4' méthoxy-4 chalcone
- trihydroxy-2',4',4 diallyl-3,3' chalcone (connu sous le nom de Kazonol)
- trihydroxy-2',4',4 (methyl-3-but-2-ène)-5' chalcone (connu sous le nom de Broussochalcone B)
- pentahydroxy-2',3',4',6',4 chalcone (connu sous le nom de Carthamin)

Parmi les dérivés de chalcone mentionnés ci-dessus, on utilisera en particulier l'hydroxy-4' chalcone répondant à la formule (Va) suivante : 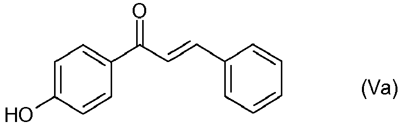 ou encore le pentahydroxy-2',3',4',6',4 chalcone (connu sous le nom de Carthamin) répondant à la formule (Vb) suivante :

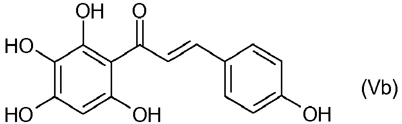

Il est connu d'utiliser ces dérivés de chalcones dans des compositions solaires, notamment dans les brevets FR 2555167, FR 2602228 et FR 2608150.

Le filtre UV organique lipophile peut être de préférence choisi parmi :
- les dérivés salicyliques, en particulier l'homosalate, l'ethylhexyl salicylate,
- les dérivés cinnamiques, tel que l'ethylhexyl methoxycinnamate,
- les dérivés de β,β'-diphénylacrylate tel que l'octocrylène,
- les dérivés de dibenzoylméthane tel que le butyl Methoxydibenzoylmethane,
- les dérivés de triazine tel que l'ethylhexyl triazone, le diethylhexyl Butamido Triazone,
- les dérivés de benzotriazole tels que le drometrizole trisiloxane,
- et leurs mélanges.

Parmi les filtres UV hydrophiles ou hydrosolubles utilisables selon l'invention, on peut les filtres suivants désignés ci-dessous sous leur nom INCI :
- les filtres hydrosolubles UVA capables d'absorber les UV de 320 à 400 nm tels que :
   - Terephthalylidène Dicamphre Acide Sulfonic Acid fabriqué sous le nom "MEXORYL SX" par CHIMEX
   - les dérivés bis-benzoazolyle tels que décrits dans les brevets EP 669 323, et US 2,463,264 et plus particulièrement le composé Disodium Phenyl Dibenzimidazole Tetra-sulfonate vendu sous le nom commercial commercial "NEO HELIOPAN AP" par Haarmann et REIMER,
- les filtres hydrosolubles UVB capables d'absorber les UV de 280 à 320 nm, tels que :
   les dérivés de p-aminobenzoïque (PABA) comme PABA, Glyceryl PABA, et PEG-25 PABA vendu sous le nom "UVINUL P25" par BASF
   Phenylbenzimidazole Sulfonic Acid vendu notamment sous le nom commercial "EUSOLEX 232" par MERCK,
   Acide férulique
   Acide salicylique
   DEA methoxycinnamate
   Benzylidène Camphre Acide Sulfonique fabriqué sous le nom "MEXORYL SL" par CHIMEX,
   Camphre Benzalkonium Methosulfate fabriqué sous le nom "MEXORYL SO" par CHIMEX, et
- les filtres hydrosolubles UVA et UVB, tels que
   Benzophenone-4 vendu sous le nom commercial " UVINUL MS40" par BASF, Benzophenone-5, et
   Benzophenone-9.

### Phase aqueuse

La phase aqueuse des compositions selon l'invention comprend au moins de l'eau. Selon la forme galénique de la composition, la quantité de phase aqueuse peut aller de 0,1 à 99 % en poids, de préférence de 0,5 à 98 % en poids, mieux de 30 à 95 % en poids, et encore mieux de 40 à 95 % en poids par rapport au poids total de la composition. Cette quantité dépend de la forme galénique de la composition désirée. La quantité d'eau peut représenter tout ou une partie de la phase aqueuse, et elle est généralement d'au moins 30 % en poids par rapport au poids total de la composition.

La phase aqueuse peut comprendre au moins un solvant hydrophile comme par exemple les mono-alcools inférieurs substantiellement linéaires ou ramifiés ayant de 1 à 8 atomes de carbone, comme l'éthanol, le propanol, le butanol, l'isopropanol, l'isobutanol ; les polyols tels que le propylène glycol, l'isoprène glycol, le butylène glycol, le propylène glycol, le glycérol, le sorbitol, les polyéthylènes gycols et leurs dérivés, et leurs mélanges.

### Phase grasse

La proportion de la phase grasse de l'émulsion peut aller par exemple de 1 à 80 % en poids, de préférence de 2 à 50 % en poids, et mieux de 5 à 30% en poids par rapport au poids total de la composition.
Cette quantité indiquée ne comprend pas la teneur en filtre lipophiles.

La nature de la phase grasse (ou phase huileuse) de l'émulsion n'est pas critique. La phase grasse peut ainsi être constituée par tous les corps gras classiquement utilisés dans les domaines cosmétique ou dermatologique, elle comprend notamment au moins une huile (corps gras liquide à 25°C.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R^{a}COOR^{b} et R^{a}OR^{b} dans laquelle R^{a} représente le reste d'un acide gras comportant de 8 à 29 atomes de carbone, et R^{b} représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;

- les hydrocarbures substantiellement linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12, le ceteareth-12 et le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1^{®}" et "FLUTEC PC3^{®}" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluorométhoxybutane vendu sous la dénomination "MSX 4518^{®}" par la Société 3M et le nonafluoroéthoxyisobutane ; les dérivés de perfluoromorpholine, tels que la 4-trifluorométhyl perfluoromorpholine vendue sous la dénomination "PF 5052^{®}" par la Société 3M ;
- les huiles de silicone comme les polyméthylsiloxanes (PDMS) volatiles ou non à chaîne siliconée substantiellement linéaire ou cyclique, liquides ou pâteux à température ambiante, notamment les cyclopolydiméthylsiloxanes (cyclométhicones) telles que la cyclohexadiméthylsiloxane et la cyclopentadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; la pâte de vaseline.

Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Les émulsionnants sont généralement présents dans la composition, en une proportion allant de 0,1 à 30 % en poids, et de préférence de 0,2 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90^{R} par la société Goldschmidt, ou le mélange polyglycéryl-4 isostéarate/cétyl diméthicone copolyol/hexyllaurate vendu sous la dénomination ABIL WE 09 par la société Goldschmidt. On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ;, et leurs dérivés oxyalkylénés ; les éthers d'alcools gras ; les éthers de sucre et d'alcools gras en C₈-C₂₄, et leurs mélanges.

Comme ester de glycéryle et d'acide gras, on peut citer notamment le stéarate de glycéryle (mono-, di- et/ou tri-stéarate de glycéryle) (nom CTFA : glyceryl stearate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stearate), le monostéarate de polyéthylène glycol 100 OE (nom CTFA: PEG-100 stearate et leurs mélanges.

On peut aussi utiliser des mélanges de ces tensioactifs, comme par exemple le produit contenant du Glyceryl stearate et du PEG-100 stearate, commercialisé sous la dénomination ARLACEL 165 par la société Uniqema, et le produit contenant du Glyceryl stearate (mono-distéarate de glycéryle) et du stéarate de potassium, commercialisé sous la dénomination TEGIN par la société Goldschmidt (nom CTFA : glyceryl stearate SE).

Comme éthers d'alcools gras, on peut citer par exemple les éthers de polyéthylène glycol et d'alcool gras comportant de 8 à 30 atomes de carbone, et notamment de 10 à22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Selon un mode de réalisation particulier, la composition comprend au moins un émulsionnant choisis parmi les inulines modifiées hydrophobes.
Par 'inuline modifiée hydrophobe' selon l'invention, on entend notamment une inuline modifiée par des chaînes hydrophobes, en particulier modifiée par greffage de chaînes hydrophobes sur le squelette hydrophile de ladite inuline.

L'inuline appartient à la famille des fructanes essentiellement linéaires dont les unités fructose sont pour la plupart liées par des liaisons β-2-1.

L'inuline peut être obtenue par exemple à partir de chicorée, de dahlia ou de topinambours. De préférence, l'inuline utilisée dans la composition selon l'invention est obtenue par exemple à partir de chicorée.
Les inulines, utilisées dans les compositions selon l'invention sont modifiées hydrophobes. En particulier, elles sont obtenues par greffage de chaînes hydrophobes sur le squelette hydrophyle du fructane.
Les chaînes hydrophobes susceptibles d'être greffées sur la chaîne principale du fructane peuvent notamment être des chaînes hydrocarbonées linéaires ou ramifiées, saturées ou insaturées, ayant de 1 à 50 atomes de carbone, telles que les groupements alkyle, arylalkyle, alkylaryle, alcoylène ; des groupements divalents cycloaliphatiques ou des chaînes organopolysiloxanes. Ces chaînes hydrocarbonées ou organopolysiloxanes peuvent notamment comprendre une ou plusieurs fonctions ester, amide, uréthane, carbamate, thiocarbamate, urée, thio-urée, et/ou sulfonamide tels que notamment méthylènedicyclohexyl et isophorone ; ou des groupements divalents aromatiques tels que phénylène.
En particulier, l'inuline présente un degré de polymérisation de 2 à environ 1000 et de préférence de 2 à environ 60, et un degré de substitution inférieur à 2 sur la base d'une unité fructose.

Selon un mode préféré de réalisation, les chaînes hydrophobes présentent au moins un groupement alkyle carbamate de formule R-NH-CO- dans laquelle R est groupement alkyle ayant de 1 à 22 atomes de carbone.
Selon un mode plus préféré de réalisation, les chaines hydrophobes sont des groupements lauryle carbamate.
En particulier, à titre illustratif et non limitatif des inulines modifiées hydrophobes pouvant être utilisées dans les compositions selon l'invention, on peut citer la stéaroyl inuline telle que celles vendues sous les dénominations Lifidrem INST par la société Engelhard et Rheopearl INS par la société Ciba ; la palmitoyl inuline ; l'undécylénoyl inuline telle que celles vendues sous les dénominations Lifidrem INUK et Lifidrem INUM par la société Engelhard ; et l'inuline lauryl carbamate tel que celui vendu sous la dénomination INUTEC SP1 par la société ORAFTI.
En particulier, on utilise une inuline greffée lauryl carbamate, notamment issue de la réaction d'isocyanate de lauryle sur une inuline, en particulier issue de la chicorée. A titre d'exemple de ces composés, on peut notamment citer le produit vendu sous la dénomination INUTEC SP1 par la société ORAFTI.
Le taux d'inuline modifiée hydrophobe dans la composition de l'invention peut aller de 0.01 à 20% en poids, de préférence de 0,01 à 10% en poids , de préférence de 0,05 à 10% en poids, en particulier de 0,1 à 10% en poids, et de préférence de 0.1 à 5% en poids et plus préférentiellement encore de 0,1 à 1% en poids (de matière active) par rapport au poids total de ladite composition.

De façon connue, toutes les compositions de l'invention peuvent contenir un ou plusieurs des adjuvants habituels dans les domaines cosmétique et dermatologique, des agents gélifiants et/ou épaississants hydrophiles ou lipophiles ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des agents filmogènes ; des matières colorantes (pigments tels que les oxydes de fer et le dioxyde de titane), nacres, colorants solubles), des charges ; et leurs mélanges.

Les quantités de ces différents adjuvants sont celles classiquement utilisées dans les domaines considérés. En particulier, les quantités d'actifs varient selon le but recherché et sont celles classiquement utilisées dans les domaines considérés, et par exemple de 0,1 à 20 %, et de préférence de 0,5 à 10 % du poids total de la composition.

Comme gélifiants hydrophiles autres que les polymères décrits ci-dessus, on peut citer par exemple les polymères carboxyvinyliques tels que les carbopols (carbomers) et les Pemulen (Copolymère acrylate/C₁₀-C₃₀-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les dérivés cellulosiques tels que l'hydroxyéthylcellulose ; les polysaccharides et notamment les gommes telles que la gomme de xanthane ; et leurs mélanges.

Comme gélifiants lipophiles, on peut citer les argiles modifiées telles que le l'hectorite et ses dérivés, comme les produits commercialisés sous les noms de Bentone.

### Actifs

Comme indiqué ci-dessus, la composition de l'invention est stable en présence d'actifs hydrophiles sensibles à l'oxydation et permet de stabiliser de tels actifs. Selon l'invention, on entend par « actif hydrophile », un composé ayant une solubilité dans l'eau d'au moins 0,25 % à température ambiante (25°C). En outre, selon l'invention, on entend par « actif hydrophile sensible à l'oxydation » tout actif d'origine naturelle ou synthétique susceptible de subir une dégradation par un mécanisme d'oxydation. Ce phénomène d'oxydation peut avoir plusieurs causes, en particulier la présence d'oxygène, de lumière, d'ions métalliques, une température élevée, ou encore certaines conditions de pH.

On peut citer à titre d'exemple d'actif hydrophile sensible à l'oxydation, et de façon non limitative, l'acide ascorbique et ses dérivés tels que le 5,6-di-O-diméthylsilylascorbate (vendu par la Sté Exsymol sous la référence PRO-AA), le sel de potassium du dl-alpha-tocopheryl-21-ascorbyl-phosphate (vendu par la Société Senju Pharmaceutical sous la référence SEPIVITAL EPC), l'ascorbyl phosphate de magnésium, l'ascorbyl phosphate de sodium (vendu par la Société Roche sous la référence Stay-C 50) ; le phloroglucinol ; les enzymes ; et leurs mélanges. Parmi les actifs hydrophiles sensibles à l'oxydation, on utilise selon un mode de réalisation préféré de l'invention l'acide ascorbique. L'acide ascorbique peut être de toute nature. Ainsi, il peut être d'origine naturelle sous forme de poudre ou sous forme de jus d'orange de préférence concentré. Il peut être aussi d'origine synthétique, de préférence sous forme de poudre.

Comme autres actifs utilisables dans la composition de l'invention, on peut citer par exemple les agents hydratants tels que les hydrolysats de protéines et les polyols comme la glycérine, les glycols comme les polyéthylène glycols,; les extraits naturels ; les anti-inflammatoires ; les oligomères procyannidoliques ; les vitamines comme la vitamine A (rétinol), la vitamine E (tocophérol), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants comme les fibres ; les agents tenseurs ; et leurs mélanges.

Outre les filtres solaires (ou filtres U.V.) organiques, la composition selon l'invention peut comprendre en outre des filtres UV inorganiques qui sont des particules d'oxyde métallique ayant une taille moyenne de particule élémentaire inférieure ou égale à 500 nm, plus préférentiellement comprise entre 5 nm et 500 nm, et encore plus préférentiellement comprise entre 10 nm et 100 nm, et préférentiellement entre 15 et 50 nm. Ils peuvent être notamment choisis parmi les oxydes de titane, de zinc, de fer, de zirconium, de cérium ou leurs mélanges. Selon leur caractère lipophile, ou au contraire hydrophile, plus ou moins prononcé, les filtres minéraux pourront être présents soit dans la phase grasse de l'émulsion, soit dans la phase aqueuse, ou bien même encore dans les deux phases à la fois.

Les compositions conformes à la présente invention peuvent comprendre en outre des filtres UV organiques insolubles comportant au moins un groupe absorbant le rayonnement UV peuvent être choisis notamment parmi les filtres UV organiques insolubles de type oxalanilide, amide vinylique, cinnamide, benzazole, benzofuranne, arylvinylidène-cétone, amide d'acrylonitrile, sulfonamide d'acrylonitrile, carbamate d'acrylonitrile ou phénylène-bis-benzoxazinone.

Comme charges qui peuvent être utilisées dans la composition de l'invention, on peut citer par exemple, les pigments tels que les oxydes de titane, de zinc ou de fer et les pigments organiques ; le kaolin ; la silice ; le talc ; le nitrure de bore ; les poudres sphériques organiques, les fibres ; et leurs mélanges. Comme poudres sphériques organiques, on peut citer par exemple les poudres de polyamide et notamment les poudres de Nylon® telles que Nylon-1 ou Polyamide 12, commercialisées sous les dénominations ORGASOL par la société Atochem ; les poudres de polyéthylène ; le Téflon® ; les microsphères à base de copolymères acryliques, telles que celles en copolymère diméthacrylate d'éthylène glycol/ methacrylate de lauryle commercialisées par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch. Comme fibres, on peut citer par exemple les fibres de polyamide, telles que notamment les fibres de Nylon 6 (ou Polyamide 6) (nom INCI : Nylon 6), de Nylon 6,6 (ou Polyamide 66) (Nom INCI : Nylon 66) ou telles que les fibres de poly-p-phénylène téréphtamide ; et leurs mélanges. Ces charges peuvent être présentes dans des quantités allant de 0 à 20 % en poids et de préférence de 0,5 à 10 % en poids par rapport au poids total de la composition.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.
Les compositions selon l'invention peuvent se présenter sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un mode préféré de réalisation de l'invention, la composition se présente sous forme d'une émulsion et notamment d'une émulsion H/E.
En outre, les compositions utilisées selon l'invention peuvent être plus ou moins fluides et avoir l'aspect d'un gel, d'une crème blanche ou colorée, d'une pommade, d'un lait, d'une lotion, d'un sérum, d'une pâte, d'une mousse.

La composition présente de préférence un pH qui respecte la peau et qui va généralement de 3 à 8 et de préférence de 4,5 à 7.

L'invention a aussi pour objet l'utilisation cosmétique d'une composition cosmétique telle que définie ci-dessus, comme produit de protection solaire (protection contre le soleil et/ou les U.V. des appareils à bronzer).

Les exemples qui suivent permettront de mieux comprendre l'invention, sans toutefois présenter un caractère limitatif. Les quantités indiquées sont en % en poids, sauf mention contraire.

### Exemples 1 à 3 : Emulsions H/E hydratantes SPF 15

On prépare une composition selon l'invention (exemple 1) comprenant un filtre lipophile et un polymère superabsorbant et deux compositions comparatives (exemples 2 et 3) comprenant un filtre solaire lipophile et des polymères acryliques qui ne sont pas superabsorbants:

| Phase | | Exemple 1 (invention) | Exemple 2 (comparatif) | Exemple 3 (comparatif) |
|---|---|---|---|---|
| A | eau | qsp 100 | qsp 100 | qsp 100 |
| | conservateurs | qs | qs | qs |
| B | Isononanoate d'isononyle | 8 | 8 | 8 |
| | Butyl Methoxydibenzoylmethane | 3 | 3 | 3 |
| | (« PARSOL 1789 » de HOFFMANN LA ROCHE) | | | |
| | Octocrylène (UVINUL N539 » par BASF) | 7 | 7 | 7 |
| | Ethylhexyl Salicylate (« NEO HELIOPAN OS » de SYMRISE | 5 | 5 | 5 |
| | Inuline lauryl carbamate (INUTEC SP1 de ORAFTI) | 0.3 | 0.3 | 0.3 |
| C | Glycérine | 3 | 3 | 3 |
| | Microsphères de polyacrylates réticulés à 89% en matières actives en mélange avec de la silice dans eau (AQUA KEEP 10 SH NF de Sumitomo Seika) | 0.5 | - | - |
| | Polyacrylate de sodium réticulé à 90% en matières sèches dans eau (Cosmedia SP de COGNIS) | - | - | 1 |
| | Homopolymère acrylique à 98 % en matières sèches dans eau (Carbopol 980 de Lubrizol) | | 0.5 | |
| | Hydroxyde de sodium | | 0.2 | |

### Mode opératoire

Chauffer la phase A jusqu'à 85°C puis revenir à 75°C
Sous mixeur, ajouter la phase B dans la phase A à 75°C.
Sous agitation au Rayneri, ajouter la phase C dans le mélange (A+B), puis laisser refroidir jusque température ambiante sous agitation Rayneri.

On obtient trois gel-crèmes de viscosités comparables.

La composition de l'exemple 1 selon l'invention est douce et fraîche à l'application, sans aucun effet collant ni en cours d'application ni après la pénétration du produit dans la peau, contrairement aux compositions des exemples 2 et 3 qui laissent un film gras et collant sur la peau.

### Exemples 4 et 5 : Emulsions H/E solaires SPF 30

On prépare une composition selon l'invention (exemple 4) comprenant un filtre lipophile et un polymère superabsorbant et une composition comparative (exemple 5) comprenant un filtre solaire lipophile et un polymère acrylique qui n'est pas superabsorbant :

| Phase | | Exemple 4 (invention) | Exemple 5 (comparatif) |
|---|---|---|---|
| A | Eau | qsp 100 | 56.64 |
| | Propylene glycol | 6 | 6 |
| | Glycérine | 6 | 6 |
| | Potassium cetyl phosphate (AMPHISOL K de DSM NUTRITIONAL PRODUCTS) | 1 | 1 |
| | EDTA | 0.1 | 0.1 |
| | ACIDE B-B'CAMPHOSULFONIQUE [1-4 DIVINYLBENZENE] EN SOLUTION AQUEUSE à 33% en matières actives (MEXORYL SX CHIMEX) | 0.9 | 0.9 |
| | Triethanolamine | 0.16 | 0.16 |
| | conservateurs | qs | qs |
| Phase B | Acide stéarique | 1 | 1 |
| | Mélange mono/disterate de glyceryle / stearate de polyethylene glycol (100 OE) (Arlacel 165 FL de Croda) | 1 | 1 |
| | C12-15 ALKYL BENZOATE (TEGOSOFT TN d'EVONIK GOLDSCHMIDT) | 7.5 | 7.5 |
| | Butyl Methoxydibenzoylmethane (« PARSOL 1789 » de HOFFMANN LA ROCHE) | 3 | 3 |
| | Octocrylène (UVINUL N539 de BASF) | 7 | 7 |
| | Ethylhexyl triazone (UVINUL T150 de BASF) | 0.5 | 0.5 |
| | Tocophérols (DECANOX MTS 50 ADM) | 0.1 | 0.1 |
| | Triéthanolamine | 0.3 | 0.3 |
| C | Oxyde de titane rutile traité aluminium | 3 | 3 |
| | stearate/alumine (MICRO TITANIUM DIOXIDE MT-100 T V de TAYCA) | | |
| D | Isohexadécane | 2 | 2 |
| | Microsphères de polyacrylates réticulés à 89% en matières actives en mélange avec de la silice dans eau (AQUA KEEP 10 SH NF de Sumitomo Seika) | 0.5 | - |
| | Homopolymère acrylique à 98 % en matières sèches dans eau (Carbopol 980 de Lubrizol) | - | 0.5 |
| | Hydroxyde de sodium | | 0.2 |
| | Gomme de xanthane | 0.1 | 0.1 |
| E | Ethanol | 2 | 2 |

### Mode opératoire

Chauffer la phase A jusqu'à 85°C puis revenir à 75°C.
Sous mixeur, ajouter la phase B dans la phase A à 75°C.
Sous agitation au Rayneri, disperser la phase C dans le mélange (A+B), puis ajouter la phase D, puis la phase E, toujours sous agitation, puis laisser refroidir jusque température ambiante sous agitation Rayneri.

La composition de l'exemple 4 selon l'invention est douce et fraîche à l'application, sans aucun effet collant ni en cours d'application ni après la pénétration du produit dans la peau, contrairement à la composition de l'exemple 5 qui laisse un toucher poisseux à l'application sur la peau et un film collant après pénétration.

## Revendications

1. Composition cosmétique sous forme d'émulsion comprenant au moins une phase aqueuse et au moins une phase grasse, un polymère superabsorbant choisi parmi les polyacrylates de sodium réticulés ayant un diamètre moyen en nombre inférieur ou égal à 100 µm et se présentant sous forme de particules sphériques et au moins un filtre UV organique.

2. Composition selon la revendication 1, **caractérisée en ce que** le polymère superabsorbant présente une capacité d'absorption d'eau allant de 20 à 2000 fois son propre poids de préférence de 30 à 1500 et mieux de 50 à 1000.

3. Composition selon l'une des revendications précédentes, **caractérisée en ce que** le polymère superabsorbant est présent en une teneur en matière active allant de 0,03 à 15 % en poids, de préférence de 0,05 à 10% en poids, de préférence allant de 0,1 à 5 % en poids, préférentiellement allant de 0,1 à 3%, voire de 0,1 à 2 % en poids par rapport au poids total de la composition.

4. Composition selon l'une des revendications précédentes, **caractérisée en ce qu'**elle comprend au moins un filtre UV organique lipophile.

5. Composition selon la revendication précédente, **caractérisée en ce que** le filtre solaire organique lipophile est choisi parmi les dérivés de l'acide para-aminobenzoique, les dérivés salicyliques, les dérivés cinnamiques, les benzophénones et aminobenzophénones, les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines, les dérivés de diphényl butadiène malonates ou malonitriles, les chalcones et leurs mélanges.

6. Composition selon l'une des revendications 4 ou 5, **caractérisée en ce que** le filtre solaire organique lipophile est choisi parmi :
- les dérivés salicyliques, en particulier l'homosalate, l'ethylhexyl salicylate,
- les dérivés cinnamiques, tel que l'ethylhexyl methoxycinnamate,
- les dérivés de β,β'-diphénylacrylate tel que l'octocrylène,
- les dérivés de dibenzoylméthane tel que le butyl methoxydibenzoylmethane,
- les dérivés de triazine tel que l'ethylhexyl triazone, le diethylhexyl butamido triazone,
- les dérivés de benzotriazole tel que le drometrizole trisiloxane,
- et leurs mélanges.

7. Composition selon l'une des revendications précédentes, **caractérisée en ce qu'**elle comprend au moins un filtre UV organique hydrophile.

8. Composition selon la revendication précédente, **caractérisée en ce que** le filtre UV organique hydrophile est choisi parmi le terephthalylidène dicamphre acid sulfonic acid, les dérivés bis-benzoazolyle, les dérivés de p-aminobenzoïque, le phenylbenzimidazole sulfonic acid, l'acide férulique, l'acide salicylique, le DEA méthoxycinnamate, le benzylidène camphre acide sulfonique, le camphre benzalkonium methosulfate, la benzophenone-4, la benzophenone-5, et la benzophenone-9.

9. Composition selon l'une des revendications précédentes, **caractérisée en ce que** les filtres organiques sont présents en des proportions allant de 0,05 à 30% en poids par rapport au poids total de la composition, de préférence allant de 0,1 à 20% en poids, et mieux de 0,5 à 15% en poids par rapport au poids total de la composition.

10. Procédé de traitement cosmétique d'une matière kératinique, **caractérisé par le fait qu'**on applique sur la matière kératinique, une composition cosmétique telle que définie selon l'une quelconque des revendications 1 à 9.

## Patentansprüche

1. Kosmetische Zusammensetzung in Form einer Emulsion, die mindestens eine wässrige Phase und mindestens eine Fettphase, ein superabsorbierendes Polymer, ausgewählt aus vernetzten Natriumpolyacrylaten, die einen zahlenmittleren Durchmesser von weniger als oder gleich 100 µm besitzen und in Form kugelförmiger Teilchen vorliegen, und mindestens einen organischen UV-Filter umfasst.

2. Zusammensetzung nach Anspruch 1, **dadurch gekennzeichnet, dass** das superabsorbierende Polymer ein Wasserabsorptionsvermögen des 20- bis 2000-Fachen seines Eigengewichts, vorzugsweise des 30- bis 1500-Fachen und besser des 50- bis 1000-Fachen aufweist.

3. Zusammensetzung nach einem der vorhergehenden Ansprüche, **dadurch gekennzeichnet, dass** das superabsorbierende Polymer in einem Wirkstoffgehalt von 0,03 bis 15 Gew.-%, vorzugsweise von 0,05 bis 10 Gew.-%, vorzugsweise von 0,1 bis 5 Gew.-%, vorzugsweise von 0,1 bis 3 Gew.-%, sogar von 0,1 bis 2 Gew.-%, bezogen auf das Gesamtgewicht der Zusammensetzung, vorliegt.

4. Zusammensetzung nach einem der vorhergehenden Ansprüche, **dadurch gekennzeichnet, dass** sie mindestens einen lipophilen organischen UV-Filter umfasst.

5. Zusammenfassung nach dem vorhergehenden Anspruch, **dadurch gekennzeichnet, dass** der lipophile organische UV-Filter aus para-Aminobenzoesäurederivaten, Salicylsäurederivaten, Zimtsäurederivaten, Benzophenonen und Aminobenzophenonen, Anthranilderivaten, Dibenzoylmethanderivaten, β,β-Diphenylacrylatderivaten, Benzylidenkampferderivaten, Phenylbenzimidazolderivaten, Benzotriazolderivaten, Triazinderivaten, Bisresorcinyltriazinen, Imidazolinderivaten, Benzalmalonatderivaten, 4,4-Diarylbutadienderivaten, Benzoxazolderivaten, Merocyaninen, Diphenylbutadienmalonat- oder -malonitrilderivaten, Chalkonen und deren Gemischen ausgewählt ist.

6. Zusammensetzung nach einem der Ansprüche 4 oder 5, **dadurch gekennzeichnet, dass** der lipophile organische UV-Filter aus den Folgenden ausgewählt ist:
- Salicylsäurederivaten, insbesondere Homosalat, Ethylhexylsalicylat,
- Zimtsäurederivaten, wie Ethylhexylmethoxycinnamat,
- β,β'-Diphenylacrylatderivaten, wie Octocrylen,
- Dibenzoylmethanderivaten, wie Butylmethoxydibenzoylmethan,
- Triazinderivaten, wie Ethylhexyltriazon, Diethylhexylbutamidotriazon,
- Benzotriazlderivaten, wie Drometrizoltrisiloxan
- und deren Gemischen.

7. Zusammensetzung nach einem der vorhergehenden Ansprüche, **dadurch gekennzeichnet, dass** sie mindestens einen hydrophilen organischen UV-Filter umfasst.

8. Zusammensetzung nach dem vorhergehenden Anspruch, **dadurch gekennzeichnet, dass** der hydrophile organische UV-Filter aus Terephthalylidendikampfersulfonsäure, Bisbenzoazylolderivaten, p-Aminobenzoesäurederivaten, Phenylbenzimidazolsulfonsäure, Ferulasäure, Salicylsäure, DEA-Methoxycinnamat, Benzylidenkampfersulfonsäure, Kampferbenzalkoniummethosulfat, Benzophenon-4, Benzophenon-5 und Benzophenon-9 ausgewählt ist.

9. Zusammensetzung nach einem der vorhergehenden Ansprüche, **dadurch gekennzeichnet, dass** die organischen Filter in Anteilen von 0,05 bis 30 Gew.-%, bezogen auf das Gesamtgewicht der Zusammensetzung, vorzugsweise von 0,1 bis 20 Gew.-% und besser von 0,5 bis 15 Gew.-%, bezogen auf das Gesamtgewicht der Zusammensetzung, vorliegen.

10. Verfahren zur kosmetischen Behandlung einer Keratinsubstanz, **dadurch gekennzeichnet, dass** man auf die Keratinsubstanz eine kosmetische Zusammensetzung nach einem der Ansprüche 1 bis 9 aufbringt.

## Claims

1. Cosmetic composition in the form of an emulsion comprising at least one aqueous phase and at least one fatty phase, a superabsorbent polymer chosen from crosslinked sodium polyacrylates having a number-average diameter of less than or equal to 100 µm and provided in the form of spherical particles, and at least one organic UV screening agent.

2. Composition according to Claim 1, **characterized in that** the superabsorbent polymer exhibits a water-absorbing capacity ranging from 20 to 2000 times its own weight, preferably from 30 to 1500 and better still from 50 to 1000.

3. Composition according to either of the preceding claims, **characterized in that** the superabsorbent polymer is present in a content as active material ranging from 0.03 to 15% by weight, preferably from 0.05 to 10% by weight, preferably from 0.1 to 5% by weight, preferentially from 0.1 to 3% by weight, indeed even from 0.1 to 2% by weight, with respect to the total weight of the composition.

4. Composition according to one of the preceding claims, **characterized in that** it comprises at least one lipophilic organic UV screening agent.

5. Composition according to the preceding claim, **characterized in that** the lipophilic organic sunscreen is chosen from para-aminobenzoic acid derivatives, salicylic derivatives, cinnamic derivatives, benzophenones or aminobenzophenones, anthranilic derivatives, dibenzoylmethane derivatives, β,β-diphenylacrylate derivatives, benzylidenecamphor derivatives, phenylbenzimidazole derivatives, benzotriazole derivatives, triazine derivatives, bisresorcinyltriazines, imidazoline derivatives, benzalmalonate derivatives, 4,4-diarylbutadiene derivatives, benzoxazole derivatives, merocyanines, diphenylbutadienemalonate or -malonitrile derivatives, chalcones and their mixtures.

6. Composition according to either of Claims 4 and 5, **characterized in that** the lipophilic organic sunscreen is chosen from:
- salicylic derivatives, in particular homosalate or ethylhexyl salicylate,
- cinnamic derivatives, such as ethylhexyl methoxycinnamate,
- β,β-diphenylacrylate derivatives, such as octocrylene,
- dibenzoylmethane derivatives, such as butyl methoxydibenzoylmethane,
- triazine derivatives, such as ethylhexyl triazone or diethylhexyl butamido triazone,
- benzotriazole derivatives, such as drometrizole trisiloxane,
- and their mixtures.

7. Composition according to one of the preceding claims, **characterized in that** it comprises at least one hydrophilic organic UV screening agent.

8. Composition according to the preceding claim, **characterized in that** the hydrophilic organic UV screening agent is chosen from terephthalylidene dicamphor sulfonic acid, bisbenzoxazolyl derivatives, p-aminobenzoic derivatives, phenylbenzimidazole sulfonic acid, ferulic acid, salicylic acid, DEA methoxycinnamate, benzylidene camphor sulfonic acid, camphor benzalkonium methosulfate, benzophenone-4, benzophenone-5 and benzophenone-9.

9. Composition according to one of the preceding claims, **characterized in that** the organic screening agents are present in proportions ranging from 0.05 to 30% by weight, with respect to the total weight of the composition, preferably ranging from 0.1 to 20% by weight and better still ranging from 0.5 to 15% by weight, with respect to the total weight of the composition.

10. Method for the cosmetic treatment of a keratinous substance, **characterized in that** a cosmetic composition as defined according to any one of Claims 1 to 9 is applied to the keratinous substance.
